# EUROPEAN PATENT APPLICATION

(11) **EP 4 844 771 A2**
(43) Date of publication of application: **25.11.2026**
(21) Application number: 26202327.8
(22) Date of filing: 17.05.2023
(51) Int. Cl.: G16B 40/10

(54) **A COMPUTER CONTROLLED METHOD FOR ANALYZING MOLECULAR TIME SERIES DATA, A RELATED SYTEM AND RELATED DEVICES**

(30) Priority: 17.05.2022 BE 202205373
(62) Document number(s) of the earlier application(s) in accordance with Art. 76 EPC: 23727535.9 / 4 526 888
(71) Applicant: UGENTEC, 1000 Brussel (BE)
(72) Inventor: VREYSEN, Samme, 1000 Brussel (BE); MAILLEUX, Jo, 1000 Brussel (BE)
(74) Representative: Gevers Patents

(57) **Abstract**

The invention provides a computer controlled method and related device for interpreting molecular time series data said method comprising the steps of acquiring, a plurality of discrete measurements of data in time on the presence of molecular fragments to generate a molecular time series data and interpolating, said plurality of discrete measurements of data in time of said molecular time series data to obtain an interpolated molecular time series data comprising a spacing of an equal Euclidean distance between said interpolated discrete measurements of data in time, rotating said interpolated molecular time series data comprising said interpolated plurality of discrete measurements of data in time in discrete steps, calculating, a distribution of said interpolated discrete measurements of data in time of said interpolated molecular time series data, in each dimension of said interpolated molecular time series data for each discrete rotation step and detecting, a characteristic phase at occurrence of a subset of said interpolated discrete measurements of data in time wherein said subset of interpolated measurements are distributed substantially in one dimension, for each said discrete step of rotation.

## Description

### Technical field

The present invention relates to a method for analyzing molecular time series data, a related system and related devices.

### Background art

Molecular biology revolves around the granularity level of nucleotides or amino acids. When measurements on molecular fragments are performed at successive points in time, molecular time series data is produced. Hence Polymerase Chain Reaction (PCR), Mass spectrometry (MS), Loop-mediated isothermal amplification (LAMP), Melt curve and High Resolution Melt (HRM) are examples of techniques that produce molecular time series data that can be analyzed for detection, identification, typing or fingerprinting of biological analytes (e.g. human DNA or micro-organisms).

Humans are particularly good at interpreting molecular time series data as the mammalian visual system is primed to respond strongly to line-shaped objects in a coarse-to-fine fashion over time (Hubel and Wiesel (1962), Bredfeldt and Ringach (2002), Vreysen et al. (2012)). A lab technician is trained to recognize specific patterns and phases independent of acquisition differences (hardware) or proprietary analysis methods (software).

Moreover, the lab technician is capable of reasoning about certain anomalies or deviations and overstep these to make conclusions about the underlying pattern.

Current molecular data analysis techniques often rely on mathematical modelling to derive information and conclusions from time series data. These models can only take that much variation and anomalies into account as it was explicitly conceptualized for and will fail otherwise.

Other approaches comprise machine learning techniques in an attempt to generalize the data problem, but with the drawback of being inheritably a black-box method with limited explainability.

Polymerase Chain Reaction, further referred to as PCR, is such a technique that generates molecular time series data. PCR rapidly makes billions of copies of specific DNA fragments for genetic testing or identification of infectious agents (e.g. virus or bacteria). Direct analysis of isolated DNA fragments is nearly impossible without PCR amplification because significant amounts are needed to be detectable by molecular and genetic analysis techniques.

Specific DNA fragments are amplified in a series of cycles of temperature changes that mimic real-life biological processes. After each cycle, the DNA fragments have (theoretically) doubled. Starting with a single fragment of DNA and doubling it 40 times results in about 100 billion copies. Specifically chosen short single-stranded DNA fragments or 'primers' that target a specific location in the DNA are used ensuring only the desired DNA fragment is amplified. By adding small fluorescent probes to these primers the amount of DNA can be detected by a specific device called a cycler instrument. These fluorescent probes only emit light upon excitation and when bound to DNA. This fluorescent signal becomes stronger as more DNA copies are amplified with every temperature cycle over time.

Such a PCR test results in a signal further referred to as a PCR amplification curve, in which a PCR amplification comprises the amount of fluorescence, which represents the amount of molecular fragments, such as DNA fragments, measured at a plurality of discrete moments in time.

This amplification curve hence is shaped by the amount of fluorescence that is generated during the PCR test. In case the specific DNA fragment is little or not present in the sample, the curve will be flat. However, if the specific DNA fragment is present, a typical PCR curve (a sigmoidal shape or S-curve) is generated.

Such curve hence starts flat as a baseline, since not sufficient DNA has been amplified to generate a signal strong enough to be detected. Once sufficient DNA has been amplified, the signal rises above the detection limit of the device and increases exponentially each cycle. This characteristic phase is further referred to as the exponential phase. As the reaction continues, more reaction components are consumed and amplification starts to slow down. This characteristic phase is further referred to as the linear phase. Eventually the reaction comes to a stop as components become fully depleted and the increase of fluorescent signal stagnates, which marks the last phase: the plateau phase.

Correctly identifying and interpreting these characteristic phases is essential in providing a clinical result to the patient.

Currently, in order to identify such characteristic phases in the acquired PCR curve, approaches often use a mathematical fitting in an attempt to identify one or more PCR curve characteristic phases to correctly and automatically interpret a PCR curve. An example of this would be the logistic fit to approximate the sigmoidal shaped curve or an estimation or identification of the baseline for baseline-rotation and -subtraction. Baseline-rotation accounts for any fluorescent drift over the course of the PCR reaction while subtraction removes any background fluorescence.

However, fitting methodologies have caveats, specifically when the PCR signal is not as clean as these models require. Many factors influence the PCR kinetic reaction introducing variation and anomalies causing these algorithms to fail. Some examples are sample handling (e.g. bad DNA extraction, pipetting errors), unbalanced reconstitution of lyophilized reactions, inhibitors present in the samples, cycler calibration, multiplexing challenges, primer-dimers, etc.

Hence current fitting methodologies in view of the current applied approaches experience shortcomings for optimal detection of and identification of the different characteristic phases (baseline, exponential, linear and plateau phase) in in molecular time series data.

### Disclosure of the invention

It is an objective of embodiments of the present invention to provide a method for robustly detecting and identifying the different characteristic patterns of phases in molecular time series data, a related molecular time series processing device of the above known type but wherein the aforementioned shortcoming or drawbacks of the known solutions are alleviated or overcome.

This objective is achieved by rotating said molecular time series data, comprising said plurality of data points where the plurality of data points being located on said curve defined by said a molecular time series data acquired, in discrete rotation steps in the Euclidean space, the plurality of data points located on said curve defined by said a molecular time series data acquired, can be better aligned with potential candidate phases such as the horizontal or vertical characteristic phase in case of two dimensional molecular time series data. In this manner the detection of various potential candidate characteristic phases within the molecular time series data is facilitated as no specific pattern or attempts to fit a mathematical function is required and as a consequence this method is significantly more robust to any variation, exception or deviation of the acquired signal.

This step of rotating is succeeded by calculating, by means of the computer processing means, a distribution of the data points of said molecular time series data, in each dimension of said molecular time series data for each discrete rotation step in the Euclidean space and subsequently the computer processing means is configured to detect, for each discrete step of rotation in the Euclidean space, a candidate characteristic phase at occurrence of a subset of said discrete measurements of data in time wherein said data points of said subset of measurements are distributed or congregate substantially along one dimension. In case of two-dimensional data, the measurements are distributed or congregate horizontally or vertically.

A further relevant embodiment relates to the computer controlled method for analyzing molecular time series data comprises the step of first interpolating, by a computer processing means, said plurality of discrete measurements of data in time of said molecular time series data acquired, where said molecular time series data comprises a plurality of discrete measurements in time on the presence of molecular fragments present in a sample, by adding interpolation data points, at a spacing of an equal Euclidean distance between said interpolation data points, on said curve of said molecular time series data to generate interpolated molecular time series data comprising a plurality of interpolation data points having a spacing of an equal Euclidean distance between said interpolation data points, said plurality of interpolation data points being located on said curve as defined by said a molecular time series data acquired.

This step of interpolating is performed in order to optimize the distribution of the samples in each dimension and consequently to better and more accurately capture the curve characteristics from the molecular time series data forming a signal indicating the amount of molecule fragments being present.

Interpolating is the adding of interpolation data points having a spacing of an equal Euclidean distance between each of said interpolation data points, on the curve that is defined by the discrete measurements of the molecular time series data and as a consequence does the interpolated molecular time series data comprise interpolation data points along the shape defined by the discrete measurements of data in time acquired by the data measurement means where said interpolation data points having a spacing of an equal Euclidean distance between said interpolation data points.

An equal Euclidean distance is an equal distance in the Euclidean space between each of the subsequent interpolated data points along the shape as defined by the discrete measurements as acquired.

Each data measurement having a certain value of presence of such molecular fragments as the luminance is measured where this luminance is a measure for the amount of molecular fragments at a certain moment in time in a sample. The curve is generated by using the luminance value, being the indication of the amount of molecular fragments being present, and the moment of time of each corresponding data measurement. A characteristic phase of the molecular time series of data is a directional linear stretch of data points in one of the dimensions. In the context of two dimensional data, this may be a vertical, inverted vertical and horizontal phase in the plurality of (interpolation) data points having a spacing of an equal Euclidean distance between said (interpolation) data points, where the (interpolation) data points of such a subset of the plurality (interpolation) data points are congregated substantially along one dimension only.

In other words, either the measured data points or the (interpolation) data points of such a subset of the plurality (interpolation) data point are located approximately or substantially on a line along one dimension, in case of two dimensional data e.g. along a horizontal or a vertical dimension.

Examples of such molecular time series data are PCR amplification data or alternatively Mass spectrometry (MS), Loop-mediated isothermal amplification (LAMP), Melt curve and High Resolution Melt (HRM) data.

A further relevant embodiment relates to the computer controlled method for analyzing molecular time series data comprises the step of transforming by said computer processing means, said plurality of values of presence of said molecular fragments in said plurality of discrete measurements of data in time lying in a first range, into values of said presence of said molecular fragments in said plurality of discrete measurements of data in time having a second range, for improving the detection of linear fragments within said plurality of discrete measurements of data in time.

The object of the transformation is to better enable the detection of linear fragments within said plurality of discrete measurements of data in time.

As an example, the exponential phase of a qPCR signal can be logarithmic transformed to result in a detectable linear vertical phase.

In this embodiment such value of presence of said molecular fragments in said plurality of discrete measurements may be a luminance value being an indication of the number of molecular fragments present in a sample.

The step of transforming said plurality of values of presence of said molecular fragments in said plurality of discrete measurements of data in time may include the step of normalizing said plurality of values of presence of said molecular fragments in said plurality of discrete measurements of data in time.

A further particular relevant embodiment of the present invention comprises the step of aggregating each said characteristic phase of said molecular time series of data detected, by concatenating each detected characteristic phase along time to generate a pattern comprising a sequence of concatenated detected characteristic phases.

Such concatenation of the different characteristic phases along time creates a pattern where these different, detected characteristic phases are concatenated in a sequence which sequence is based on the time and/or based on the predetermined sequence of the (interpolation) data points of such a subset of the plurality (interpolation) data points within the plurality of (interpolation) data points.

Specific logic can be applied for each of the different possible patterns including at least subsets of functionally relevant patterns. Based on recognition of such patterns a classification system can be performed that optionally in addition also applies functional domain knowledge in this step of classification.

At first, a linear phase is detectable as there is a high number of samples detectable in the vertical dimension, a baseline can be detected as a high number of samples is detected in the horizontal dimension before the linear phase and finally as there is a further relative high number of samples found in a horizontal dimension after the linear phase, the plateau phase can be deduced from this number of samples.

Still another relevant embodiment of the present invention comprises the analyzing of the pattern of sequences of concatenated detected characteristic phases generated for classifying said pattern of sequences of concatenated characteristic phases which analysis optionally is performed based on additional features of said measured signal. For each characteristic phase, specific logic can be applied to create additional characteristic phase specific relevant metrics. In addition, the sequence of the different detected characteristic phases, creates a classification system which can be utilized in following steps as context or to perform specific logic for a given class of data.

For each specific phase, depending on the type of phase, additional context can be created by calculating relevant metrics for that specific phase (e.g. the regression through the horizontal baseline phase in PCR curve is used to project the curve so that the baseline is set around zero).

As an example, in polymerase chain reaction PCR data these detected characteristic phases translate to the baseline, exponential, linear phase and plateau phase of a PCR amplification curve. The outcome of this method can be used as input in classification models, including machine learning based models, which provide an interpretation of the time series.

This classification further may be based on further additional features of the signal calculated from the measured signal, i.e. the plurality of discrete measurements of data in time on the presence of molecular fragments. These features are calculated based on the measured signal, i.e. the plurality of discrete measurements of data in time on the presence of molecular fragments.

Examples of such additional features of the signal calculated are the noise level, relative fluorescence range, amplification efficiency, position of anomalies, Cq, and end fluorescence.

These features can be part of a model to generate an outcome. This model may be based on and not limited to machine learning or on expert AI which leverages domain knowledge. In qPCR the outcome is a classification of having an either positive, negative or inconclusive amplification signal.

Furthermore, the method according to the present invention relates to the generation representative features of molecular time series data that is resistant to anomalies or deviations in the measurement signal. These features closely resemble the patterns the human brain would identify and process to come to a conclusion about the signal. Hence, our novel approach enhances the explainability.

Still another relevant embodiment of the present invention is that the Molecular series data processing device MSDPD further comprises a data measurement means MEME that is configured to acquire a plurality of discrete measurements of data in time on the presence of molecular fragments to generate a molecular time series data.
This embodiment relates to a device comprising all essential means for performing the analysis interpretation of the molecular time series data according to the present invention incorporated in a single device.

Still another relevant embodiment of the present invention is that the Molecular series data processing device MSDPD further comprises a reception means RM that is configured to receive from a separate, stand alone, data measurement device DMD said molecular time series data comprising said plurality of discrete measurements of data in time on the presence of molecular fragments

Still another relevant embodiment of the present invention is that Molecular series data processing device MSDPD further comprises an output means OM configured to output, e.g. display for presenting relevant information such as any inputs, final and/or intermediate results etc.

A further relevant embodiment of the present invention is the data measurement device DMD that comprises a measurement means MEME that is configured to acquire, a plurality of discrete measurements of data in time on the presence of molecular fragments, to generate a molecular time series data, said plurality of data measurements in time, defining a certain curve.

Another relevant embodiment of the present invention is that the Molecular series A data measurement device DMD further comprises a data forwarding means DFM that is configured to forward said molecular time series data comprising a plurality of discrete measurements of data in time on the presence of molecular fragments towards a Molecular series data processing device.

A further relevant embodiment of the present invention is Server device that comprises the Molecular series data processing device MSDPD according to the present invention.

### Brief description of the drawings

The invention will be further elucidated by means of the following description and the appended figures:
Fig. 1 represents the Molecular series data processing device MSDPD for interpreting molecular time series data in combination with the functional elements of the molecular series data processing device wherein the Molecular series data processing device MSDPD is implemented in a remote server RS coupled to a user measurement device DMD according to embodiments of the present invention.

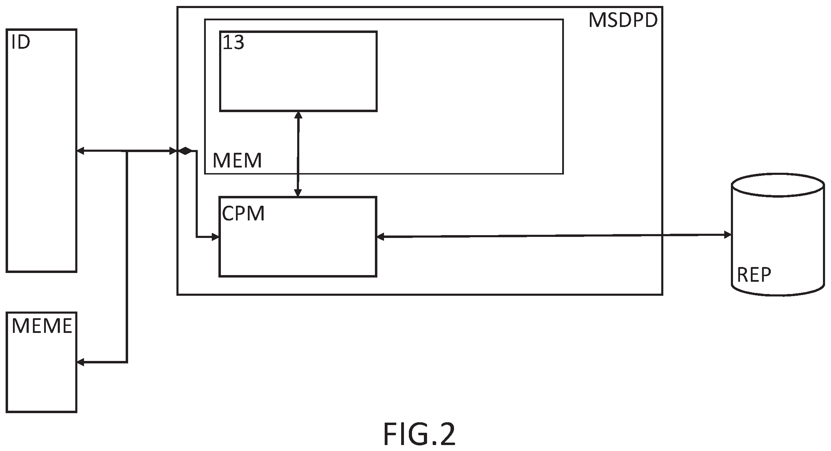

Fig. 2 represents the Molecular series data processing device MSDPD for interpreting molecular time series data with the functional means of the molecular series data processing device according to embodiments of the present invention.

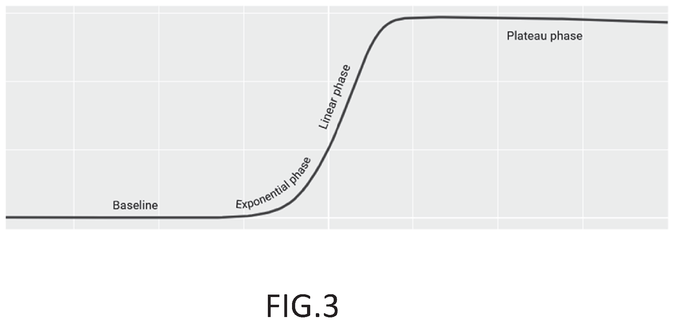

Fig. 3 represents an example of a molecular time series data (e.g. PCR amplification curve) for analysis according to embodiments of the present invention.
Fig. 4A - 4C represent respectively the acquired signal (4A), the acquired signal which is interpolated comprising a plurality of interpolation data points with equal Euclidean distance between each data point (4B) and the acquired signal which is interpolated and rotated by 3 example angles (4C).
Fig. 5 represents the acquired and interpolated signal at a given rotation together with the distribution of the samples along the different dimensions, identifying the characteristic phases of the molecular time series data signal.

### Modes for carrying out the invention

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

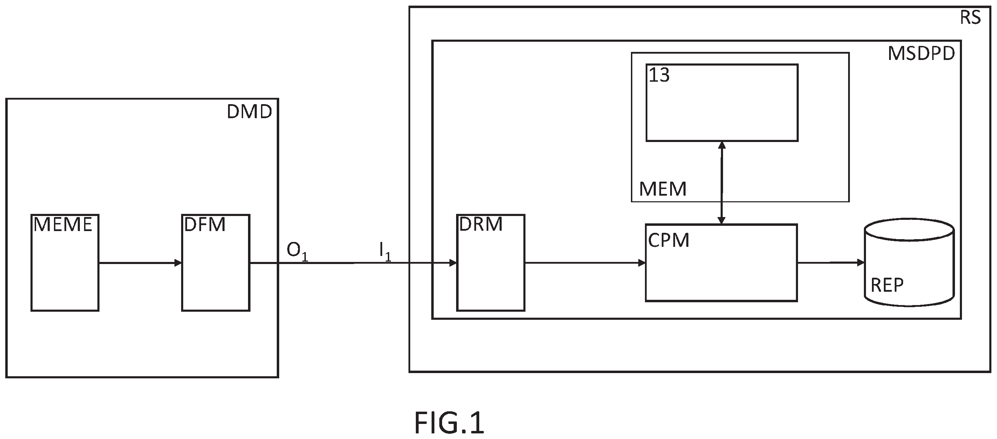

In the following paragraphs, referring to the drawing in FIG.1, an implementation of the molecular series data processing system for interpreting molecular time series data according to an embodiment of the present invention is described.

In a further paragraph, all connections between mentioned elements are defined.

Subsequently all relevant functional means of the system for interpreting molecular time series data as presented in FIG.1 are described followed by a description of all interconnections.

In the succeeding paragraph the actual execution of the interpreting molecular time series data according to an embodiment of the present invention under control of the system is described. A first essential element of this system is the molecular series data processing device MSDPD being a separate device like a computing device being standalone or implemented in a remote server device. Alternatively the molecular series data processing device MSDPD may be incorporated in such standalone computing device or a in a remote device like a remote server RS. The molecular series data processing device MSDPD may alternatively form part of or be implemented by a cycler instrument device, a mass spectrometer, or a plate reader.

A first essential element of the molecular series data processing device MSDPD for interpreting molecular time series data according to an embodiment of the present invention is the computer processing means CPM for executing a software application for interpreting molecular time series data according to an embodiment of the present invention which computing device may be implemented at a distributed server system or at a local standalone device such as a computing device. Alternatively, the computer processing means CPM could be implemented at a cycler instrument device, a mass spectrometer or plate reader. The molecular series data processing device MSDPD further may comprise an internal memory MEM for storing programs, intermediate results and final results of the actual execution of the interpreting molecular time series.

A second relevant element of the molecular series data processing device processing device MSDPD is the memory MEM for storing the software application for interpreting molecular time series data. The memory is configured to store at least one further application for being executed by the computer processing means CPM. Such a memory MEM may be a local computing memory device but alternatively may be an external computing memory and optionally a distributed external computing memory. This computer processing means CPM may be implemented by means of a computer microprocessor. The molecular series data processing device processing device MSDPD may further comprise a data reception means DRM that is configured to receive from said data measurement device DMD said molecular time series data comprising said plurality of discrete measurements of data in time on the presence of molecular fragments.

In the alternative, the molecular series data processing device processing device MSDPD further may instead of the data reception means DRM comprise a data measurement means MEME for acquiring a plurality of discrete measurements of data in time on the presence of molecular fragments to generate a molecular time series data. This data measurement means MEME for acquiring a plurality may alternatively be (as shown) be incorporated in a separate device, i.e. the data measurement device DMD for being coupled to the molecular series data processing device processing device MSDPD. The data reception means may be a (radio) digital signal receiver and the forwarding means may be a (radio) digital signal transmitter.

The measurement means MEME of the data measurement device DMD is coupled to the data forwarding means DFM that in turn is coupled to an output-terminal O₁ of the data measurement device DMD.

The data reception means DRM of the Molecular series data processing device MSDPD first has an input terminal I₁ that is at the same time an input terminal of the molecular series data processing device processing device MSDPD. The reception means further is coupled to the processing means CPM that is turn is coupled with an input/output to an input/output of the repository. Moreover, the processing means CPM is coupled with an input/output to an input/output of the memory MEM.

The processing means CPM for interpreting molecular time series data (resulting from measured molecular data, is configured to first obtain a molecular time series data comprising a plurality of discrete measurements of data in time on the presence of molecular fragments from an incorporated data reception means DRM of the molecular series data processing device processing device MSDPD or from the data measurement device DMD. The processing means CPM further optionally is configured to subsequently interpolate said plurality of discrete measurements of data in time of said molecular time series data by adding interpolation data points on said curve of molecular time series data to generate an interpolated molecular time series data which comprises a plurality of interpolation data points having a spacing of an equal Euclidean distance between said interpolation data points, having a spacing of an equal Euclidean distance between each of the interpolation data points, where said plurality of interpolation data points being located on said curve defined by said molecular time series data as acquired.

The optional step of interpolating is followed by the step of rotating the interpolated molecular time series data, comprising the plurality of interpolation data points having a spacing of an equal Euclidean distance between each of the interpolation data points, said plurality of interpolation data points being located on said curve defined by said a molecular time series data as acquired, in discrete rotation steps in the Euclidean space and subsequently calculate a distribution of said plurality of interpolation data points of said interpolated molecular time series data, in each dimension of said interpolated molecular time series data for each discrete rotation step in the Euclidean space and finally detect, for each said discrete step of rotation the Euclidean space, characteristic phases of said molecular time series of data at occurrence of a subset of said interpolated molecular time series data, which comprises the plurality of interpolation data points having a spacing of an equal Euclidean distance between each of the interpolation data points, where the plurality of interpolation data points being located on said curve defined by said a molecular time series data as acquired, wherein said subset of interpolation data points are distributed substantially along one (e.g. vertical or horizontal) dimension.

The molecular series data processing device MSDPD may be coupled with a user interfacing device ID, having a user interface comprising a display for displaying measured inputs, intermediate and final results of the actual execution of the interpreting molecular time series etc., in combination with all tools and options for interfacing with the computing device to manage and control all actions involved in the test for interpreting molecular time series data such as controlling the inputting of measurements, the processing of the measurements and the controlling thereof. The user interfacing device ID further may comprise an input means such as a keyboard and/or a mouse for interacting with the molecular series data processing device MSDPD and a display for presenting relevant information such as any inputs, final and/or intermediate results etc.

The interaction may include rendering measured inputs, intermediate and final results of the actual execution of the interpreting molecular time series etc.

A third relevant element of the system is a repository REP, for e.g. storing results of the measured inputs, intermediate and final results of the actual execution of the interpreting molecular time series etc. This repository REP, may be a local database or be a distributed storage system or any suitable alternative system or device.

This memory, for example maintained by the server, e.g. a database can be accessed by all relevant parties for managing measurements, etc.

The molecular series data processing device MSDPD may form part of a computing system like a workstation as a desktop personal computer or a laptop personal computer or equally suitable device or be implemented at a server device being located locally or remotely from the user e.g. enabling the application of web-based or cloud based interpreting molecular time series data application and or access thereto and corresponding measured input-data, intermediate and final results of the actual execution of the interpreting molecular time series data etc. , in combination with all tools and options for interfacing and managing the applications.

Furthermore, in an alternative embodiment of the present invention, there is a measurement means MEME that forms part of the molecular series data processing device MSDPD or is implemented as an external measurement device, for acquiring a plurality of discrete measurements of data in time on the presence of molecular fragments to form or generate a molecular time series data. This measurement means MEME may be implemented by a cycler instrument device, a plate reader or a mass spectrometer.

Finally, the Molecular series data processing device MSDPD further comprises an output means OM configured to output the detected characteristic phases of the acquired or measured input-signal, i.e. the molecular time series data etc.

A second relevant element of this Molecular series data processing system is a data measurement device DMD. This data measuring device DMD comprises a measurement means MEME that is configured to acquire a plurality of discrete measurements of data in time on the presence of molecular fragments generating a molecular time series data. This plurality of discrete data measurements in time defining a certain curve.

This data measuring device DMD further comprises a data forwarding means DFM, which is configured to forward said molecular time series data comprising a plurality of discrete measurements of data in time on the presence of molecular fragments towards a molecular series data processing device MSDPD. This forwarding may be performed via certain kind of data carrier like or a hard-disk or alike but may also be performed via a communications link, including data transmitter and data receiver, between the molecular series data processing device processing device MSDPD and the data measurement device DMD.

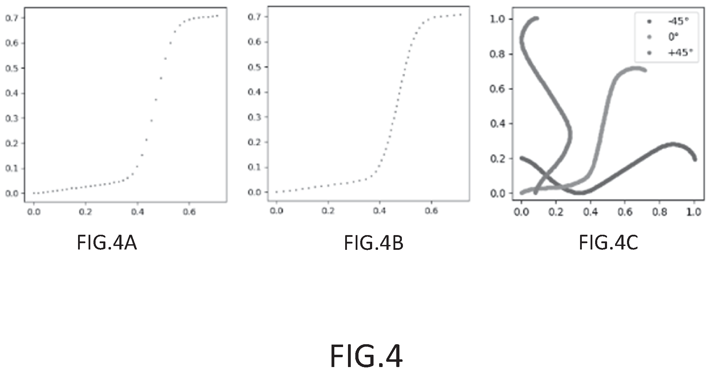

In order to explain an embodiment of the present invention it is assumed that a molecular time series data is acquired where the molecular time series data comprises a plurality of measurements of a process like e.g. a polymerase chain reaction or alternatively a Mass spectrometry (MS), a Loop-mediated isothermal amplification (LAMP), Melt curve or a High Resolution Melt (HRM) process being executed. As a result a signal is acquired comprising a plurality of discrete measurements of data in time on the presence of molecular fragments to generate a molecular time series data as is shown in Fig. 4A.

In order *to explain embodiment* of the present invention it is further assumed that this - molecular time series data is acquired by means of the data measurement device DMD that comprises a measurement means MEME which acquired molecular time series data comprising a plurality of discrete measurements of data in time on the presence of molecular fragments by means of a data forwarding means DFM is forwarded towards a molecular series data processing device MSDPD for analyzing these acquired molecular time series data.

In molecular data analysis, a lab technician is trained to recognize specific patterns and phases which lead to a certain outcome of the analysis. Due to acquisition differences (hardware), proprietary analysis methods (software), a single human intuition based approach is pressing.

In order to provide a method for robustly detecting and identifying the different characteristic patterns of phases in molecular time series data, and wherein the aforementioned shortcoming or drawbacks of the known solutions are alleviated or overcome and a related molecular series data processing device, in a first step, the acquired signal, comprising the plurality of discrete measurements of data in time on the presence of molecular fragments may as an optional step be pre-processed by transforming this acquired molecular time series data and interpolating this acquired signal, i.e. the molecular time series data comprising a plurality of discrete measurements of data in time on the presence of molecular fragments where this plurality of discrete data measurements in time having a certain value of presence of said molecular fragments, defining a certain curve meaning that a plurality of interpolation data points is added on said curve of said molecular time series data acquired, to generate an interpolated molecular time series data which hence comprises a plurality of interpolation data points having a spacing of an equal Euclidean distance between said interpolation data points, hence resulting in a spacing between subsequent samples with equal Euclidean distance from each other as is shown in Fig. 4B. The computer processing means CPM generates an interpolation of the plurality of discrete measurements of data in time of said molecular time series data acquired, to obtain an interpolated molecular time series data comprising a spacing of an equal Euclidean distance between said interpolated discrete measurements of data in time in order to optimize the distribution of the samples in each dimension and consequently to better and more accurately capture the shape characteristics from the molecular time series data forming a signal indicating the amount of molecule fragments being present. In other words such an interpolated molecular time series data is obtained by adding on said curve of said molecular time series data acquired, instead of the original measured samples, interpolation data points having a spacing of an equal Euclidean distance between said interpolation data points, to generate an interpolated molecular time series data which hence comprises a plurality of interpolation data points having a spacing of an equal Euclidean distance between said interpolation data points.

In a second step, the interpolated signal, being the interpolated molecular time series data is rotated in the Euclidean space in discrete steps and the distribution in each dimension is calculated for each rotation step as is shown in Fig 4C.

By subsequently rotating, by means of the computer processing means, the interpolated molecular time series data comprising said plurality of interpolation data points having a spacing of an equal Euclidean distance between said interpolation data points, where said plurality of interpolation data points being located on said curve, in discrete steps in the Euclidean space, the plurality of interpolation data points of the interpolated molecular time series data can be better aligned with potential candidate phases such as horizontal characteristic phases (baseline, plateau phase) and vertical characteristic phases (linear phase) where the detection of various potential candidate phases within the molecular time series data is facilitated and is further advantageous that in embodiments of the present invention no specific pattern or attempts to fit a mathematical function is required and as a consequence is significantly more robust to any variation, exception or deviation of the acquired signal.

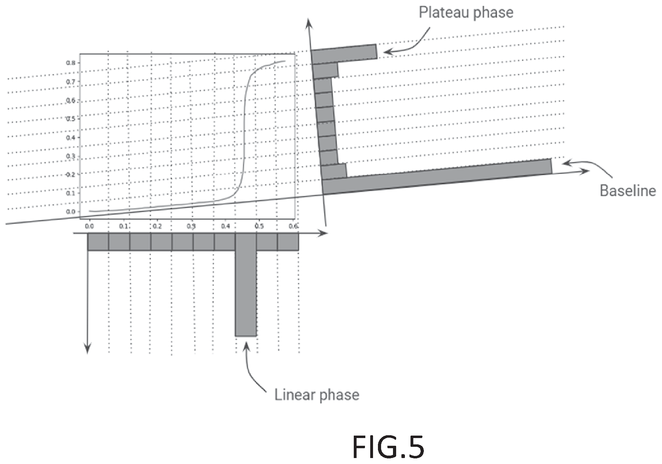

This step of rotation is succeeded by calculating, by means of the computer processing means, a distribution of the interpolated discrete measurements of data in time of said interpolated molecular time series data, in each dimension of said interpolated molecular time series data for each discrete rotation step. The computer processing means subsequently may detect a candidate characteristic phase at occurrence of a subset of said interpolated discrete measurements of data in time wherein said subset of interpolated measurements are distributed substantially in one, (vertical or horizontal) dimension for each said discrete step of rotation as is shown in Fig. 5. In case at a certain discrete rotation step in the Euclidean space the occurrence of a subset of said interpolation data points of said interpolated molecular time series data wherein said subset of interpolation data points is distributed substantially along one dimension, either in a horizontal or in a vertical direction, is detected as is shown in Fig. 4 wherein for the "baseline" a subset of interpolation data points is detected and for the "Plateau phase" wherefore a second subset of interpolation data points is detected from the plurality of interpolation data points of the interpolated molecular time series. At a further rotation angle, a third characteristic phase, "the linear phase" is detected wherefore a third subset of interpolation data points is detected from the plurality of interpolation data points of the interpolated molecular time series as is also shown in Fig. 5. In the distribution as shown in Fig. 5, each of the above mentioned characteristic phases will be represented by the highest peak in the distribution along one of the axis along all discrete rotation steps.

In other words, this step detects candidate phases as a subset of interpolation data points which are distributed closely to each other along one dimension at a specific rotation. In a two dimensional space, this means that horizontal and vertical trending phases can be detected as is shown in Fig. 5.

At first, a linear phase is detectable as there is a high number of samples detectable in the vertical dimension, a baseline can be detected as a high number of samples is detected in the horizontal dimension before the linear phase and finally as there is a further relative high number of samples found in a horizontal dimension after the linear phase, the plateau phase can be deduced from this number of samples.

Furthermore, the computer processing means CPM may additionally perform the step of _transformation by said computer processing means values of said presence of said molecular fragments in said plurality of discrete measurements of data in time lying in a first range into values of said presence of molecular fragments in said plurality of discrete measurements of data in time lying in a second range, for improving the detection of linear fragments within said plurality of discrete measurements of data in time.

The object of this step of transforming values of said presence of said molecular fragments in said plurality of discrete measurements of data in time is to better enable to detect linear fragments within said plurality of discrete measurements of data in time.

As an example, the exponential phase of a qPCR signal can be logarithmic transformed to result in a detectable linear vertical phase.

In this embodiment such value of presence of said molecular fragments in said plurality of discrete measurements may be a luminance value being an indication of the number of molecular fragments present in a sample.

It is further to be noted that the *embodiment* of the present invention is explained including the optional step of interpolating said plurality of discrete measurements of data in time of said molecular time series data, by adding interpolation data points on said curve of said molecular time series data to generate an interpolated molecular time series data although this is a non-essential step.

The step of transforming said plurality of values of presence of said molecular fragments in said plurality of discrete measurements of data in time may include the step of normalizing said plurality of values of presence of said molecular fragments in said plurality of discrete measurements of data in time.

In this manner due to this meant transformation characteristic phases are transformed to linear stretches that can be characterized using the present invention.

Furthermore, the computer processing means CPM may additionally perform the step of aggregating each said characteristic phase (of said molecular time series of data) detected along time, by concatenating each detected characteristic phase to generate a pattern comprising a sequence of concatenated detected characteristic phases.

Such concatenation of the different characteristic phases creates a pattern. Specific logic can then be applied for each of the different possible patterns including at least subsets of functionally relevant patterns. Based on recognition of such patterns a classification system can be based on (functional) domain knowledge.

At first, a linear phase is detectable as there is a high number of samples detectable in the vertical dimension, a baseline can be detected as a high number of samples is detected in the horizontal dimension before the linear phase and finally as there is a further relative high number of samples found in a horizontal dimension after the linear phase, the plateau phase can be deduced from this number of samples.

Still another relevant embodiment of the present invention comprises the analyzing of the pattern of sequences of concatenated detected characteristic phases generated for classifying said pattern of sequences of concatenated characteristic phases. For each characteristic phase, specific logic can be applied to create additional characteristic phase specific relevant metrics. In addition, the sequence of the different detected characteristic phases, creates a classification system which can be utilized in following steps as context or to perform specific logic for a given class of data.

For each specific phase, depending on the type of phase, additional context can be created by calculating relevant metrics for that specific phase (e.g. the regression through the horizontal baseline phase in PCR curve is used to project the curve so that the data points of the baseline will be located around zero relative fluorescence.

As an example, in polymerase chain reaction PCR data these characteristic phases translates to the baseline, exponential, linear and plateau phase of a PCR amplification curve. The outcome of this method can be used as input for classification models (including machine learning based models) which provide an interpretation of the time series. For each specific phase, depending on the type of phase, additional context can be created by calculating relevant metrics for that specific phase (e.g. the regression through the horizontal baseline phase in PCR curve is used to project the curve so that the baseline is set around zero).

As an example, in polymerase chain reaction PCR data these detected characteristic phases translate to the baseline, exponential, linear phase and plateau phase of a PCR amplification curve. The outcome of this method can be used as input in classification models (including machine learning based models) which provide an interpretation of the time series.

This interpretation furthermore is based on further additional features of the signal calculated from the measured signal, i.e. the plurality of discrete measurements of data in time on the presence of molecular fragments.

These features are calculated based on the measured signal, i.e. the plurality of discrete measurements of data in time on the presence of molecular fragments.

Examples of such additional signal features calculated are the are noise level, relative fluorescence range, amplification efficiency, position of anomalies, Cq, and end fluorescence.

These features can be part of a model to generate an outcome. This model may be based on (and not limited to) machine learning or on expert AI which leverages domain knowledge. In qPCR the outcome is a classification of having an either positive, negative or inconclusive amplification signal.

As an example of the decision making of such algorithm we describe two examples of qPCR curves: the first curve is described by the following calculated functional features: low noise, no anomalies, high fluorescence range, early Cq, efficiency of 1.0 and having a baseline, exponential phase, linear phase and plateau phase.

The second curve has the following functional features: medium noise, bubble anomaly at cycle 6, low relative fluorescence range, late Cq, efficiency of 0.6 and having a baseline and linear phase.

Based on domain knowledge, the algorithm can conclude that the first curve has a positive amplification signal, while the second curve has a less pronounced amplification signal, so is called inconclusive.

The various functional means of the system can be located in a central way or in a more distributed manner, where these means may be distributed over the client devices and an optional server device. A final remark is that embodiments of the present invention are described above in terms of functional blocks. From the functional description of these blocks, given above, it will be apparent for a person skilled in the art of designing electronic devices how embodiments of these blocks can be manufactured with well-known electronic components. A detailed architecture of the contents of the functional blocks hence is not given.

While the principles of the invention have been described above in connection with specific apparatus, it is to be clearly understood that this description is merely made by way of example and not as a limitation on the scope of the invention, as defined in the appended claims.

## Claims

1. A computer controlled method for analyzing molecular time series data resulting from measured molecular data, said method comprising the steps of:
- acquiring, by a data measurement means, a plurality of discrete measurements of data in time on the presence of molecular fragments to generate a molecular time series data, said plurality of discrete data measurements in time having a certain value of presence of said molecular fragments, defining a certain curve comprising a plurality of data points each corresponding to a discrete measurement; and
- rotating, by said computer processing means, said molecular time series data comprising said plurality of data points, said plurality of data points being located on said curve, in discrete rotation steps in the Euclidean space; and
- calculating, by said computer processing means, a distribution of said data points of said molecular time series data, in each dimension of said molecular time series data for each discrete rotation step in the Euclidean space; and
- detecting, by said computer processing means, for each said discrete step of rotation in the Euclidean space, a characteristic phase of said molecular time series of data, based on an occurrence of a subset of said data points of said molecular time series data wherein data points of said subset of data points congregate substantially along one, either horizontal or vertical, dimension, said characteristic phase being one of the baseline, exponential phase, linear phase and the plateau phase.

2. A computer controlled method for analyzing molecular time series data resulting from measured molecular data according to claim 1, **CHARACTERIZED IN THAT**, said method further comprises before said step of said step of rotating said molecular time series the step of:
- interpolating, by a computer processing means, said molecular time series data comprising said plurality of data points, by adding interpolation data points on said curve of said molecular time series data to generate an interpolated molecular time series data comprising a plurality of interpolation data points having a spacing of an equal Euclidean distance between said interpolation data points, said plurality of interpolation data points being located on said curve defined by said a molecular time series data as acquired; and **in that**
- said step of rotating, by said computer processing means, comprises rotating said interpolated molecular time series data comprising said plurality of interpolated data points, said plurality of interpolated data points being located on said curve, in discrete rotation steps in the Euclidean space; and **in that**
- said step of calculating, by said computer processing means, comprises calculating a distribution of said interpolated data points of said interpolated molecular time series data, in each dimension of said molecular time series data for each discrete rotation step in the Euclidean space; and **in that**
- said step of detecting, by said computer processing means, for each said discrete step of rotation in the Euclidean space, a characteristic phase of said molecular time series of data at occurrence of a subset of said interpolated data points of said interpolated molecular time series data wherein interpolation data points of said subset of interpolation data points congregate substantially along one dimension.

3. A computer controlled method for analyzing molecular time series data according to claim 1 or claim 2, **CHARACTERIZED IN THAT** said method further comprises the step of:
- transforming, by said computer processing means, said plurality of values of presence of said molecular fragments in said plurality of discrete measurements of data in time lying in a first range into values of said presence of said molecular fragments in said plurality of discrete measurements of data in time lying in a second range, for improving the detection of linear fragments within said plurality of discrete measurements of data in time.

4. A computer controlled method for analyzing molecular time series data according to any of claims 1 to 3, wherein said method further comprises the step of:
- aggregating each said characteristic phase of said molecular time series of data detected by concatenating said each detected characteristic phase along time to generate a pattern comprising a sequence of concatenated detected characteristic phases.

5. A computer controlled method for analyzing molecular time series data according to claim 4, **CHARACTERIZED IN THAT** said method further comprises the step of:
- analyzing, said pattern comprising a sequence of concatenated detected characteristic phases generated optionally in combination with features of said measured signal, for classifying said pattern of sequences of concatenated characteristic phases.

6. Molecular series data processing system (MSDPS) for analyzing molecular time series data, said Molecular series data processing system (MSDPS) comprising:
- a data measurement means (MEME) configured to acquire, a plurality of discrete measurements of data in time on the presence of molecular fragments, to generate a molecular time series data, said plurality of data measurements in time, defining a certain curve comprising a plurality of data points each corresponding to a discrete measurement; and
- a processing means (CPM) configured to:
- rotate said molecular time series data comprising said plurality of data points , said plurality of data points being located on said curve in the Euclidean space in discrete rotation steps; and
- calculate a distribution of said data points of said molecular time series data, in each dimension of said molecular time series data for each discrete rotation step in the Euclidean space; and
- detect, for each said discrete step of rotation in the Euclidean space, a characteristic phase of said molecular time series of data, based on an occurrence of a subset of said data points wherein said subset of data points, congregate substantially along one, either horizontal or vertical dimension, said characteristic phase being one of the baseline, exponential phase, linear phase and the plateau phase.

7. Molecular series data processing system (MSDPS) for analyzing molecular time series data, according to claim 6, **CHARACTERIZED IN THAT** said processing means (CPM) further is configured to:
- interpolate said plurality of discrete measurements of data in time of said molecular time series data, by adding interpolation data points on said curve of said molecular time series data to generate an interpolated molecular time series data comprising a plurality of interpolation data points having a spacing of an equal Euclidean distance between said interpolation data points, said plurality of interpolation data points being located on said curve defined by said a molecular time series data as acquired; and **in that** said processing means (CPM) further is configured to:
- rotate said interpolated molecular time series data comprising said plurality of interpolation data points having a spacing of an equal Euclidean distance between said interpolation data points, said plurality of interpolation data points being located on said curve in the Euclidean space in discrete rotation steps; and
- calculate a distribution of said interpolation data points of said interpolated molecular time series data, in each dimension of said interpolated molecular time series data for each discrete rotation step in the Euclidean space; and
- detect, for each said discrete step of rotation in the Euclidean space, a characteristic phase of said molecular time series of data, at occurrence of a subset of said interpolation data points wherein said subset of interpolated data points, congregate substantially along one dimension.

8. Molecular series data processing device (MSDPD) for use in a system according to claim 6, wherein said Molecular series data processing device (MSDPD) comprises:
- a processing means (CPM) configured to:
- rotate said molecular time series data comprising said plurality of data points, said plurality of data points being located on said curve in the Euclidean space in discrete rotation steps; and
- calculate a distribution of said data points of said molecular time series data, in each dimension of said molecular time series data for each discrete rotation step in the Euclidean space; and
- detect, for each said discrete step of rotation in the Euclidean space, a characteristic phase of said molecular time series of data, at occurrence of a subset of said data points wherein said subset of data points, are distributed substantially along one dimension.

9. Molecular series data processing device (MSDPD) according to claim 8, **CHARACTERIZED IN THAT** said processing means (CPM) further is configured to:
- interpolate said plurality of discrete measurements of data in time of said molecular time series data, by adding interpolation data points on said curve of said molecular time series data to generate an interpolated molecular time series data comprising a plurality of interpolation data points having a spacing of an equal Euclidean distance between said interpolation data points, said plurality of interpolation data points being located on said curve defined by said a molecular time series data as acquired; and
- rotate said interpolated molecular time series data comprising said plurality of interpolation data points having a spacing of an equal Euclidean distance between said interpolation data points, said plurality of interpolation data points being located on said curve in the Euclidean space in discrete rotation steps; and
- calculate a distribution of said interpolation data points of said interpolated molecular time series data, in each dimension of said interpolated molecular time series data for each discrete rotation step in the Euclidean space; and
- detect, for each said discrete step of rotation in the Euclidean space, a characteristic phase of said molecular time series of data, based on an occurrence of a subset of said interpolation data points wherein said subset of interpolated data points, are distributed substantially along one, either horizontal or vertical, dimension, said characteristic phase being one of the baseline, exponential phase, linear phase and the plateau phase.

10. Molecular series data processing device (MSDPD) according to claim 8, **CHARACTERISED IN THAT** said data processor (CPM) further is configured to:
- transform, said plurality of values of presence of said molecular fragments in said plurality of discrete measurements of data in time lying in a first range into values of said presence of said molecular fragments in said plurality of discrete measurements of data in time lying in a second range for improving the detection of linear fragments within said plurality of discrete measurements of data in time.

11. Molecular series data processing device (MSDPD) according to claim 6 or 7, **CHARACTERISED IN THAT** said data processor (CPM) further is configured to:
- aggregate each said characteristic phase of said molecular time series of data detected by concatenating each detected characteristic phase along time to generate a pattern comprising a sequence of concatenated detected characteristic phases.

12. Molecular series data processing device (MSDPD) according to claim 8, **CHARACTERISED IN THAT** said data processor (CPM) further is configured to:
- analyze, said pattern comprising a sequence of concatenated detected characteristic phases generated optionally in combination with features of said measured signal,, for classifying said pattern of sequences of concatenated characteristic phases.

13. Molecular series data processing device (MSDPD) according to any of claims 8 to 12, **CHARACTERISED IN THAT** said Molecular series data processing device (MSDPD) further comprises:
- a data measurement means (MEME), is configured to acquire a plurality of discrete measurements of data in time on the presence of molecular fragments to generate a molecular time series data.
- .

14. A data measurement device (DMD) for use in a system according to claim 6 or 7 , wherein said measuring device comprises a measurement means (MEME) configured to acquire, a plurality of discrete measurements of data in time on the presence of molecular fragments, to generate a molecular time series data, said plurality of data measurements in time, defining a certain curve.

15. Server device comprising a Molecular series data processing device (MSDPD) according to any of claims 6 to 12.
